# EUROPEAN PATENT SPECIFICATION

(11) **EP 3 017 778 B1**
(45) Date of publication and mention of the grant of the patent: **26.07.2017**
(21) Application number: 14192326.8
(22) Date of filing: 07.11.2014
(51) Int. Cl.: A61B 17/52, A61B 17/02, A61B 17/00

(54) **Medical instrument for manipulating, in particular retracting tissue or an organ**
Medizinisches Instrument zum Manipulieren, insbesondere zum Zurückziehen von Gewebe oder einem Organ
Instrument médical permettant de manipuler, en particulier un tissu ou un organe de rétraction

(43) Date of publication of application: 11.05.2016
(73) Proprietor: University Of Dundee, Dundee DD1 4HN (GB)
(72) Inventor: Cuschieri, Alfred, St. Andrews Fife KY16 9TY (GB); Wang, Zhigang, Dundee Medipark, Dundee DD2 1FD (GB)
(74) Representative: Witte, Weller & Partner Patentanwälte mbB

(56) References cited:
- EP-A1- 1 972 283
- AU-A1- 2010 200 269
- US-A1- 2012 010 639
- US-A1- 2013 066 136

## Description

The invention relates to a medical instrument for manipulating, in particular retracting tissue or an organ in the human or animal body, comprising an elongated shaft having a distal end portion, and a working element arranged at the distal end portion of the shaft, wherein the working element has at least one magnetically acting element generating a magnetic field for manipulating the tissue or organ, wherein the working element has a plurality of magnetically acting elements each generating a magnetic field, wherein the magnetically acting elements are arranged in-line along a longitudinal working element axis of the working element.

A medical instrument of such a type is known from US 2013/0066136 A1.

In surgical operations on patients, it is often necessary to manipulate tissue or organs during the accomplishment of surgical procedures. Such manipulations can consist in putting aside an organ or tissue in order to obtain an access to deeper lying tissue or organs on which the surgical task is to be accomplished, or in retracting tissue or an organ for a subsequent dissection of tissue, for example. In this sense, manipulating can involve any kind of moving, turning, displacing and in particular retracting tissue or organs.

Surgical tasks necessitating the manipulation of tissue or organs can arise in minimally invasive surgeries as well as in open surgeries.

Conventional instruments existing for the purpose of manipulating tissue employ grasping jaws arranged at a distal end portion of the instrument shaft, wherein the jaws are operated by the motion of a handle arranged at the proximal end portion of the instrument shaft. The jaws have grasping faces opposite to each other to grasp tissue in order to manipulate the same. The use of such conventional grasping instruments for manipulating tissue or organs exhibits several drawbacks. Pressure exerted on the tissues by the jaws may cause direct trauma to the surface and internal structures of the tissue. Furthermore, since such pressures may occlude the vascular supply, the tissues may become traumatized through ischemia.

In order to overcome these drawbacks of conventional instruments, manipulating tissue or organs using magnetic forces has been conceived. US 2007/0135685 A1 discloses a medical instrument for manipulating, in particular retracting tissue or an organ in the human or animal body, which comprises an elongated shaft and at least one working element arranged at a distal end portion of the shaft, the working element comprising at least one magnetically acting element generating a magnetic field for manipulating the tissue or organ. In some embodiments disclosed therein the working element has one magnetically acting element which is rotatable about a rotation axis running through the magnetically acting element in order to adjust the strength of the magnetic force interacting between the magnetically acting element and the tissue or organ to be manipulated. In other embodiments, the medical instrument disclosed in that document has a plurality of working elements each having one magnetically acting element, wherein the plurality of working elements are arranged in parallel at the distal end portion of the shaft. Each working element can be moved with respect to the instrument shaft.

The tissue or organ to be manipulated by such a medical instrument using magnetic forces is treated with a magnetically acting substance, for example by applying a small volume of glue-based magnetic media to the tissue surface or by interstitial injection of fluids, for example ferro-fluids which can be magnetized in a magnetic field and thereby attracted by the magnetically acting element of the working element through magnetic forces.

The use of magnetic forces for manipulating tissue has the main advantage that the manipulation can be carried out with at least reduced pressure and contact between the tissue and the instrument thus avoiding or at least reducing traumata to the tissue or organ.

Further documents relating to instruments and methods for magnetic manipulation of tissue and organs are US 2007/0270629 A1, US 2007/0135679 A1, US 2007/0108860 A1, US 2008/0171907 A1, US 2010/0204727 A1, and US 2010/0168523 A1.

Despite of the afore-mentioned advantages of manipulating tissue or organs by magnetic forces, there are some restrictions and limitations in the use of magnetic force manipulation of tissue in particular in minimally invasive surgeries. In minimally invasive surgery, the instrument has to be inserted through a small diameter port like a trocar sleeve. Usually, medical instruments for use in minimally invasive surgery can have a shaft outer diameter of 10 mm or less, e.g. 5 mm, 3 mm or 2.5 mm. This implies that the magnetically acting element at the distal end portion of the instrument shaft as known from US 2007/0135685 A1 must have a diameter which is small enough in order to fit through the small diameter access port. However, in such a case the magnetic force generated by such a small magnetically acting element is insufficient for effective manipulation, in particular retraction of tissue or organs. If the magnetic force generated by the magnetically acting element is too weak, tissue or organs cannot be reliably manipulated. On the other hand, a magnetically acting element having a larger diameter than the shaft diameter of the instrument shaft would generate a sufficiently strong magnetic field, but such a magnetically acting element cannot be inserted through the access port in minimally invasive surgeries.

The same problem arises with the embodiments in US 2007/0135685 A1 which employ a plurality of working elements each having one magnetically acting element, because the magnetically acting elements are arranged on the same axial level distributed about the instrument shaft axis. US 2013/0066136 A1 (the preamble of claim 1 is based on this document) discloses a device for locating and retrieving a misplaced foreign body that has metallic characteristics. The device includes a shaft connected to a handle and a retrieval tool that is coupled to the shaft and moves between a deployed position and collapsed position. The tool has in one embodiment a plurality of magnetic elements that generate a magnetic field, wherein the magnetic field extends over a greater area in the deployed position and is of sufficient strength to attract the foreign body such that it is held in contact with the tool.

Therefore, it is an object of the present invention to provide a medical instrument for manipulating, in particular retracting tissue or an organ in the human or animal body mentioned at the outset by means of which tissue or organs can be manipulated, in particular retracted using magnetic forces in a reliable manner, even in minimally invasive or minimal access surgery.

According to the invention, this object is achieved in that the magnetically acting elements are adjustable in position along the longitudinal working element axis.

The working element of the medical instrument according to the invention forms a manipulator tip having a plurality of magnetically acting elements, for example three or more magnetically acting elements. The magnetically acting elements form an in-line arrangement along the longitudinal working element axis. Since each magnetically acting element generates a magnetic field, the magnetic attractive force generated by each magnetically acting element sums up to a total magnetic force. By virtue of arranging a plurality of magnetically acting elements in-line along the longitudinal working element axis, the total magnetic field generated can have a large extent in direction of the longitudinal working element axis, whereby the magnetically attractive area of the working element is advantageously increased, while each single magnetically acting element can be of such a small size, in particular small diameter, which would be insufficient for generating a magnetic field sufficient for generating reliable magnetic attraction forces if there was only one magnetically acting element. Thus, each magnetically acting element can be of such a small size that the working element can have an outer diameter which is small enough to fit through a minimal access port. Hence, the medical instrument according to the invention is particularly suited for use in minimally invasive or minimal access surgery.

The magnetically acting elements are adjustable in position along the longitudinal working element axis.

In this configuration, the strength of the magnetic force can be adapted according to the actual surgical task. For example, by placing the magnetically acting elements close to one another, a magnetic field, and thereby a high magnetic force can be generated concentrated along a short path in direction of the longitudinal working element axis. Vice-versa, when the magnetically acting elements are arranged with an increasing gap between the magnetically acting elements, a magnetic field or magnetic force can be generated over a longer distance along the longitudinal working element axis.

In addition to the adjustability of the position of the magnetically acting elements along the longitudinal working element axis, provision can be made of exchangeable working elements having different numbers of magnetically acting elements and/or magnetically acting elements differently distributed along the longitudinal working element axis according to the needs of the surgical task. It is also possible to provide different working elements with different shapes of the magnetically acting elements.

In a preferred refinement, the magnetically acting elements are spaced apart from one another, with a gap between adjacent magnetically acting elements.

This configuration has the advantage that the "grasping" force of the working element is not only generated by the magnetic attraction force between the magnetically acting elements and the magnetized tissue or organ, but also by an elevated friction force between the attracted tissue and the working element. For, upon attracting tissue with the magnetically acting elements, some portions of the tissue will penetrate into the gap or gaps between the magnetically acting elements, and by withdrawing the working element in direction of the longitudinal working element axis, tissue penetrated into the gaps between the magnetically acting elements is subject to an increased friction force between the tissue and the magnetically acting elements.

It is, however, also conceivable that the magnetically acting elements are arranged without a gap between the magnetically acting elements.

In this case, the number of magnetically acting elements can be increased, whereby the resulting generated total magnetic field and force is increased, too, without increasing the longitudinal dimension of the working element.

In a further preferred refinement, the magnetically acting elements are magnetized in a direction perpendicular to the longitudinal working element axis.

The direction of magnetization of the magnetically acting elements is to be understood, for dipole magnetically acting elements, as the orientation of the pole axis between the magnetic poles. In the present configuration, the pole axis is perpendicular to the longitudinal working element axis. The advantage of this configuration is that the magnetic flux density and, thus, the strength of the magnetic field and the magnetic forces generated by the magnetically acting elements is larger than in the case where the magnetically acting elements are magnetized in a direction parallel to the longitudinal working axis. The latter, however, is also a conceivable configuration of the magnetically acting elements if only lower attractive forces are required, for example for soft tissue. With perpendicular magnetization, high magnetic attractive forces can be generated which are required for manipulating tissues or organs exhibiting a higher resistance against stretching.

In a further preferred refinement, the working element has a thin rod or pin and the magnetically acting elements each have a bore through which the rod or pin passes.

In this configuration, the magnetic field generated can fully surround the magnetically acting elements so that the full circumference of the working element around the longitudinal working element axis can be used for manipulating tissue or organs by means of magnetic forces.

In another preferred configuration, the working element has a housing magnetically inactive or magnetically weaker active than the magnetically acting elements, in which the magnetically acting elements are accommodated, wherein the housing has a lateral opening which is limited in circumferential direction around the longitudinal working element axis so that the magnetically acting elements are only partially exposed.

With this configuration, the magnetic field generated is rotationally asymmetric around the working element axis. Tissue or an organ can be reliably magnetically "grasped" with the exposed area of the magnetically acting elements, thus providing a high attracting force as in the previous mentioned refinement. The present refinement has the further advantage that the magnetically inactive or magnetically weaker active housing can be used in order to facilitate the release of the working element from the tissue or organ. Thus, for manipulating, in particular retracting tissue or an organ, the working element is oriented such that the exposed area of the magnetically acting elements faces the tissue or organ, and for release or removal of the working element from the tissue or organ, the working element is rotated about the working element axis such that the housing faces the tissue or organ. Since the housing is magnetically inactive or only magnetically weak active, the working element can be easily removed from the tissue.

In a further preferred refinement, the working element is pivotably connected to the distal end portion of the shaft so that an angle of the longitudinal working element axis with respect to a longitudinal shaft axis of the shaft can be changed.

This refinement has the advantage that the maximal retraction force before detachment of the tissue from the working element can be adjusted by setting or adjusting the angle of the working element axis with respect to the instrument shaft axis. For example, when the longitudinal working element axis is parallel to the longitudinal shaft axis, and the instrument is withdrawn in direction parallel to the longitudinal shaft axis, the maximal retraction force before the working element detaches from the tissue or organ is higher than in case the working element axis is oriented by an angle of 90° with respect to the longitudinal shaft axis. Thus, the 90°-orientation of the working element can be used for releasing the "grasped" tissue from the working element, while the 0°-orientation of the working element provides maximum retraction force for retracting the tissue or organ.

In general, in a method for manipulating, in particular retracting, tissue or an organ in the human or animal body, the working element is withdrawn in a direction forming an angle with the working element axis in a range from about 0° to about 30° for manipulating the tissue or organ.

Further in general, in a method for manipulating, in particular retracting, tissue or an organ in the human or animal body, the working element is withdrawn in a direction forming an angle with the working element axis in a range from about 70° to about 110° for releasing the tissue or organ from the working element.

It is further preferred if the angle of the longitudinal working element axis with respect to the shaft axis is changeable from about 0° to at least 90°.

In this configuration, a variable maximal retraction force can be obtained by changing the angular orientation of the working element axis with respect to the shaft axis.

It is further preferred in this context, if the working element can be fixed in a set angular pivoting position, and/or the working element can be released for free pivoting movement.

When the working element is fixed in a set angular pivoting position, the maximal tissue retraction force is set to a certain value. When the working element is released for free pivoting movement, the working element can adapt its orientation during progressing retraction, for example from an approximately 90°-orientation to an almost 0°-orientation automatically, and thus the maximal tissue retracting force continuously increases with progressing retraction. The latter is particularly advantageous, because with progressing retraction of the tissue or organ the counteracting tensile stress increases, and, thus, a reliable attraction and thereby "grasping" of the tissue organ is ensured over a larger retraction distance.

In a further preferred refinement, the working element is connected to a movable grip part of a handle arranged at a proximal end portion of the shaft for pivoting the working element.

Such a handle can be designed as a conventional scissors-grip a surgeon is familiar with. Thus, pivoting the working element can be performed by a surgeon in the same way as for closing and opening a jaw of a conventional forceps or scissors.

In a further preferred refinement, the working element is rotatable about the longitudinal working element axis.

This refinement is particularly advantageous in connection with the above refinement according to which the magnetically acting elements are arranged in a housing having a lateral opening limited in circumferential direction so the magnetically acting elements are only partially exposed. In this case, namely, the working element can be rotated such that the magnetically acting elements face the tissue or organ to be manipulated in order to obtain a high attraction force, and resulting therefrom, retraction force, or the working element can be rotated such that the housing which is magnetically inactive or magnetically weaker active than the magnetically acting elements faces the tissue or organ for easily releasing or detaching the tissue organ from the working element.

In general, in a method for manipulating, in particular retracting, tissue or an organ in the human or animal body, the working element is rotated about the longitudinal working element axis such that the exposed area of the magnetically acting elements faces the tissue or organ for manipulating the tissue or organ.

Further in general, in a method for manipulating, in particular retracting, tissue or an organ in the human or animal body, the working element is rotated about the longitudinal working element axis such that the housing faces the tissue or organ for releasing the tissue or organ from the working element.

In certain embodiments, the plurality of magnetically acting elements has at least three magnetically acting elements, for example three, four, five or more magnetically acting elements.

In particular, the magnetically acting elements each preferably have a maximal size or diameter perpendicular to the working element axis which is equal to or smaller than a diameter of the instrument shaft. The magnetically active elements preferably have a size perpendicular to the working element axis of 5 mm or less, e.g. 3 mm or 2.5 mm or even less.

The magnetically acting elements can be of any type, in particular permanent magnets. The shape of the magnetically acting elements can be of any kind, in particular cylindrical, spherical or ellipsoidal, for example.

Further features and advantages emerge from the following description and the accompanying drawings.

It is to be understood that the features mentioned before and still to be explained below cannot only be taken in the combinations indicated, but also in other combinations or in isolation, without departing from the scope of the present invention.

Exemplary embodiments are shown in the drawings and are described hereinafter with reference thereto. In the drawings:
- Fig. 1: shows a medical instrument for manipulating tissue or an organ in the human or animal body in a side view with a working element according to a first embodiment;
- Fig. 1A: shows a modified embodiment of a handle of the instrument in Fig. 1 in a side view;
- Fig. 1B: shows another modified embodiment of a handle of the instrument in Fig. 1 in a side view;
- Fig. 2: shows a distal portion of the medical instrument in Fig. 1 in a side view and rotated by 90° with respect to the side view in Fig. 1 and in an enlarged scale;
- Fig. 2A: shows further details of the distal portion in Fig. 2 in a further enlarged scale;
- Fig. 3: shows the distal portion in Fig. 2 in a side view rotated by 90° with respect to Fig. 2, wherein the working element is pivoted by 90° with respect to a longitudinal shaft axis of the instrument in Fig. 1;
- Fig. 4: shows the working element of the medical instrument in Fig. 1 removed from the medical instrument and in a perspective view;
- Fig. 5: shows another embodiment of a working element for use in the medical instrument in Fig. 1 in a lateral view;
- Fig. 6: shows still another embodiment of a working element for use in the medical instrument in Fig. 1 in a perspective side view;
- Fig. 7: schematically shows magnetization characteristics of magnetically acting elements for use in the medical instrument in Fig. 1;
- Fig. 8: schematically shows an alternative of magnetization characteristics of magnetically acting elements for use in the medical instrument in Fig. 1;
- Fig. 9: shows a diagram of a magnetic flux density distribution for the magnetization characteristics in Figs. 7 and 8;
- Fig. 10: shows the medical instrument in Fig. 1 (partially shown) inserted into a body for manipulating tissue;
- Fig. 11: shows the medical instrument in Fig. 1 (partially shown) in a manipulating step;
- Fig. 12: shows a diagram of a force curve in dependence on retraction distance for a retraction of the working element in a direction parallel to the working element axis;
- Fig. 13: shows a diagram of a force curve in dependence on retraction distance for a retraction of the working element in a direction perpendicular to the working element axis;
- Fig. 14: shows a diagram of the maximal retraction force in dependence on the angle of retraction direction with respect to the working element axis;
- Fig. 15: shows a diagram showing maximal retraction forces for different retraction modes;
- Fig. 16: shows the medical instrument in Fig. 1 (partially shown) inserted into a body for manipulating tissue, the instrument having a working element corresponding to the working element in Fig. 5;
- Fig. 17: shows the instrument in Fig. 16 in a tissue manipulating step;
- Fig. 18: shows the instrument in Fig. 16 and 17 in a tissue release step;
- Fig. 19: shows a diagram of the magnetic field generated by the working element in Fig. 5; and
- Fig. 20: shows a diagram of maximal retraction forces for the working element in Fig. 5 in different operation modes.

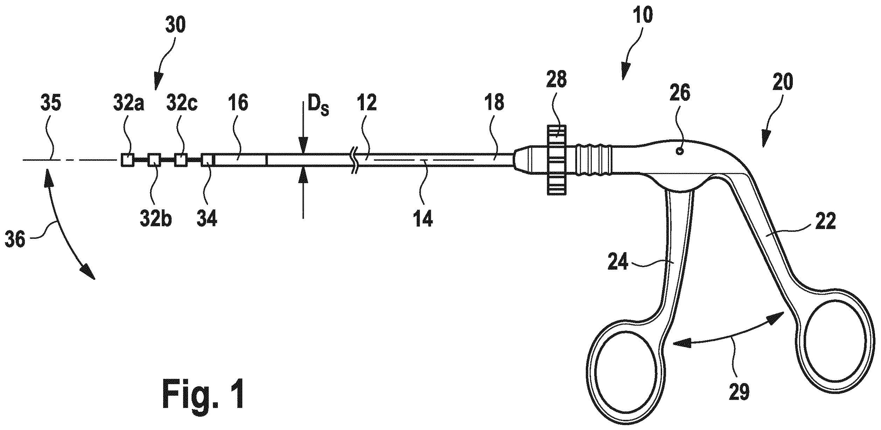

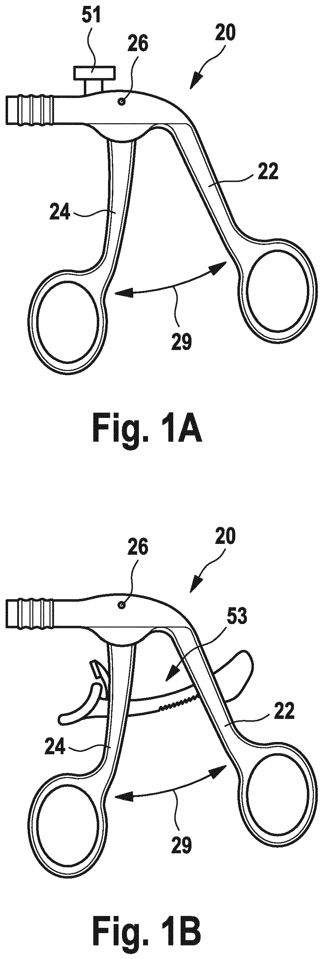

Fig. 1 shows a medical instrument for manipulating, in particular retracting tissue or an organ in the human or animal body which is labeled with general reference numeral 10. The instrument 10 is particularly, but not exclusively, suited for use in minimally invasive or minimal access surgery.

The instrument 10 comprises an elongated shaft 12 which is shown interrupted in Fig. 1 due to its large longitudinal size. The shaft 12 has a longitudinal shaft axis 14, a distal end portion 16 and a proximal end portion 18.

A handle 20 is arranged at the proximal end portion 18 of the shaft 12 and has an immovable grip part 22 and a movable grip part 24. The movable grip part 24 is pivotable about a pivot axis 26 relative to the immovable grip part 22 as indicated by a double arrow 29. Further arranged on the handle 20 is an actuating element 28 for rotating the shaft 12 about the shaft axis 14.

A working element 30 is arranged at the distal end portion 16 of the shaft 12 and connected thereto via a connector 34.

The working element 30 comprises a plurality of magnetically acting elements 32a, 32b, 32c each generating a magnetic field.

The magnetically acting elements 32a, 32b, 32c are arranged in-line along a longitudinal working element axis 35 of the working element 30. In the state of the medical instrument 10 shown in Fig. 1, the longitudinal working element axis 35 coincides with the longitudinal shaft axis 14. However, the working element 30 is pivotable with respect to the shaft 12 according to a double arrow 36 so that the working element axis 35 can be inclined with respect to the shaft axis 14.

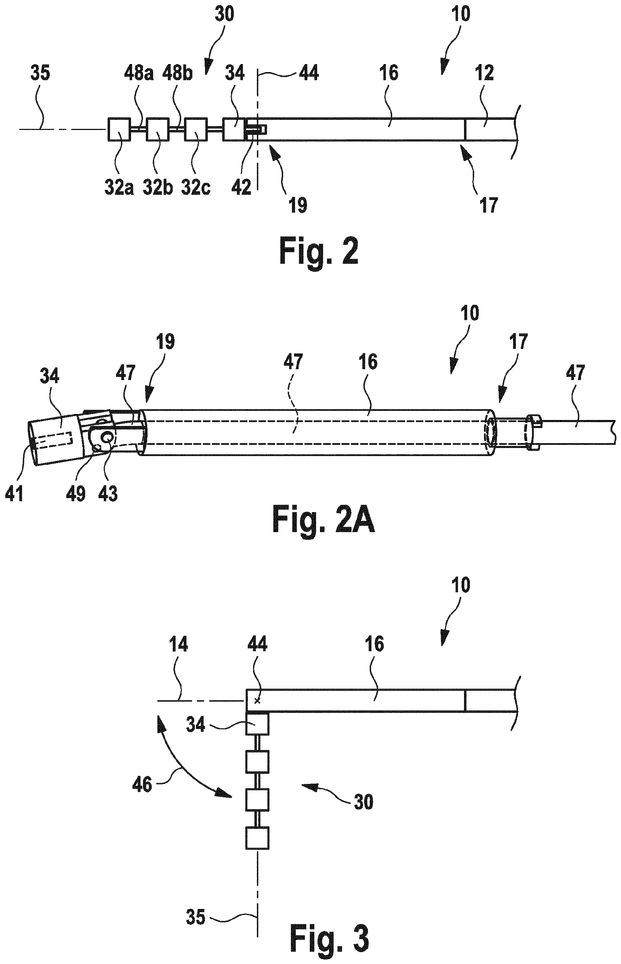

Figs. 2 and 3 show the medical instrument 10 in Fig. 1 in the region of the distal end portion 16 of the shaft 12 and of the working element 30. Fig. 2A shows further details. The distal end portion 16 is configured as a kind of a custom-design adaptor. A proximal end 17 of the distal end portion 16 is configured to fit into the shaft 12, which may be a standard instrument shaft (e.g. easy clicking fit into a Karl Storz Clickline^{®} shaft). A distal end 19 of the distal end portion or adaptor 16 is configured to provide pivotable connection to the working element 30 through the connector 34. Fig. 2A shows the distal end portion 16 with the pivotable connector 34, without the working element 30 connected to the connector 34.

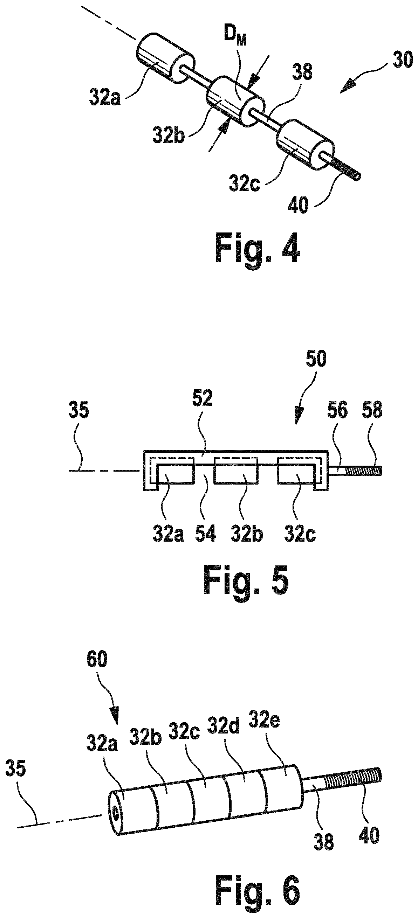

Fig. 4 shows the working element 30 in isolation. Referring to Fig. 4, the working element 30 in total has three magnetically acting elements 32a, 32b, 32c. However, it is to be understood that the number of magnetically acting elements can be lower or higher than three, for example two, four, five or more.

The magnetically acting elements 32a, 32b, 32c are configured as permanent magnets having a cylindrical shape. An outer diameter D_{M} of the magnetically acting elements 32a, 32b, 32c is equal to or lower than an outer diameter D_{S} (see Fig. 1) of the shaft 12. Instead of being cylindrical, the magnetically acting elements 32a, 32b, 32c can have any other suitable shape, for example spherical, ellipsoidal, etc.

The outer diameter D_{M} of the magnetically acting elements 32a, 32b, 32c can be as small as 5 mm or less, for example 3 mm or 2,5 mm.

In the embodiment shown in Figs. 2 to 4, the working element 30 has a thin rod or pin 38, and the magnetically acting elements 32a, 32b, 32c each have a bore through which the rod or pin 38 passes. The magnetically acting elements 32a, 32b, 32c can be fixedly or adjustably in position secured to the rod or pin 38 by any suitable means, for example by being screwed onto the pin or rod 38, or by being glued onto the pin or rod 38.

The pin or rod 38 itself is connected to the connector 34 (Fig. 2) via a screw connection, wherein to this end the pin or rod 38 has a thread 40 at its proximal end, while the connector 34 has an inner thread 41 (fig. 2A) so that the proximal end of the pin or rod 38 can be screwed into the connector 34. In this way, the working element 30 of the medical instrument 10 is exchangeable. A variety of different designs of working elements 30 having different numbers of magnetically acting elements, different surface shapes, etc. thus can be connected to the connector 34 and thus to the shaft 12 of the medical instrument 10 in an exchangeable manner.

The connector 34 itself is pivotably connected with the distal end portion 16 of the shaft 12 via a pin 43 (Fig. 2A) forming a hinge 42 defining a pivot axis 44. Through a rod shaped force transmission element 47 (Fig. 2A) connected to the connector 34 via a hinge joint pin 49 at hinge 42, this allows pivotable movement of the working element 30 about the pivot axis 44 shown in Figs. 2 and 3 to have different angles of the working element 30 with respect to the shaft 12. Fig. 3 shows the working element 30 pivoted by 90° about the pivot axis 44. A double arrow 46 in Fig. 3 shows the pivoting movement of the working element 30. The working element 30 is pivotable with respect to the shaft axis 14 by an angle in the range of about 0° to at least 90°, for example in the range of 0° to about 110°.

Pivoting of the working element 30 about the pivot axis 44 can be actuated by operating the movable grip part 24 of the instrument 10 in Fig. 1. To this end, the hinge 42 is connected to the movable grip part 24 through the force transmission element 47 (Fig. 2A) extending through the shaft 12, as known in the field of medical scissors or forceps and, thus, not described here in detail.

Displacement of the force transmission element 47 changes the angle of the working element. Further, it can be provided that the working element 30 can be fixed in any set angular pivoting position, for example in the 0° and 90° position, but it is also conceivable and preferred if the working element 30 can be released for free pivoting movement. If the working element needs to be fixed in a position at a certain angle, then a locking mechanism can be used to fix the force transmission element 47 from any movement. For example, a jaw locking knob 51 arranged at the handle 20 as shown in Fig. 1A can be used for locking the force transmission element 47 in any position, so that the angle of the working element 30 can be fixed. Such a jaw locking knob is described in the article: Frank, Cuschieri: Prehensile Atraumatic Grasper With Intuitive Ergonomics, Surgical Endoscopy 11 (1997):p. 1036-1039. Alternatively, a ratchet mechanism 53 arranged at the handle 20 as shown in Fig. 1B can be used. The ratchet mechanism 53 is configured to fix the movable grip part 24 at the immovable grip part 22 in different angular positions with respect to the immovable grip part, which is possible in a precise manner. Thus, when the movable grip part 24 is latched in a specific angular position , the working element 30 is fixed in a specific angular position with respect to the shaft 12. The ratchet mechanism 53 preferably is a disengageable ratchet mechanism. Handles with ratchet mechanisms like or similar to mechanism 103 for use in the instrument 10 are, for example, the Karl Storz Clickline^{®} ratchet handles.

Further, the working element 30 is rotatable about the working element axis 35. Such a rotational movement can be actuated by rotating the actuating element 28 (Fig. 1) or by any other suitable rotation mechanism.

In the embodiment shown in Figs. 2 through 4, the magnetically acting elements are spaced apart from one another so that a gap 48a is present between magnetically acting elements 32a and 32b, and a gap 48b is present between magnetically acting elements 32b and 32c.

Further, the magnetically acting elements 32a, 32b, 32c can be adjustable in position along the working element axis 35 which means that the gaps 48a, 48b can be made smaller, or vanish, or can be made larger.

Fig. 5 shows another embodiment of a working element 50 for use with the medical instrument 10 in Fig. 1 instead of the working element 30. Elements of the working element 50 which are identical or similar to elements of the working element 30 are labeled with the same reference numerals as for the working element 30.

The working element 50 has a housing 52 which is magnetically inactive or magnetically weaker active than the magnetically acting elements 32a, 32b, 32c. The magnetically acting elements 32a, 32b, 32c are accommodated in the housing 52 and can be fixed thereto by any suited means, or they can be adjustable in position along the working element axis 35 in the housing 52. The housing 52 has a lateral opening 54 which is limited in circumferential direction around the working element axis 35 so that the magnetically acting elements 32a, 32b, 32c are partially exposed and partially covered by the housing 52, seen in circumferential direction about the working element axis 35. The housing 52 further has a connecting pin or rod 56 having a thread 58 so that the working element 50 can be screw-connected with the connector 34 in Fig. 1.

Fig. 6 shows another embodiment of a working element 60 for use with the medical instrument 10 in Fig. 1 instead of the working element 30. Those elements of the working element 60 which are identical or similar with elements of the working element 30 are referenced with the same reference numerals.

The working element 60 has five magnetically acting elements 32a, 32b, 32c, 32d, 32e instead of three magnetically acting elements. Differently from the previous embodiments, there is no gap between the single magnetically acting elements 32a, 32b, 32c, 32d and 32e as shown in Fig. 6. In this way, the surface of the working element 60 is smooth instead of being "teethed" like the working elements 30 and 50. The magnetically acting elements 32a, 32b, 32c, 32d and 32e are arranged on the pin or rod 38 as described for the working element 30 in Fig. 4. Again, the working element 60 can be screwed into the connector 34 (Fig. 1) through the thread 40.

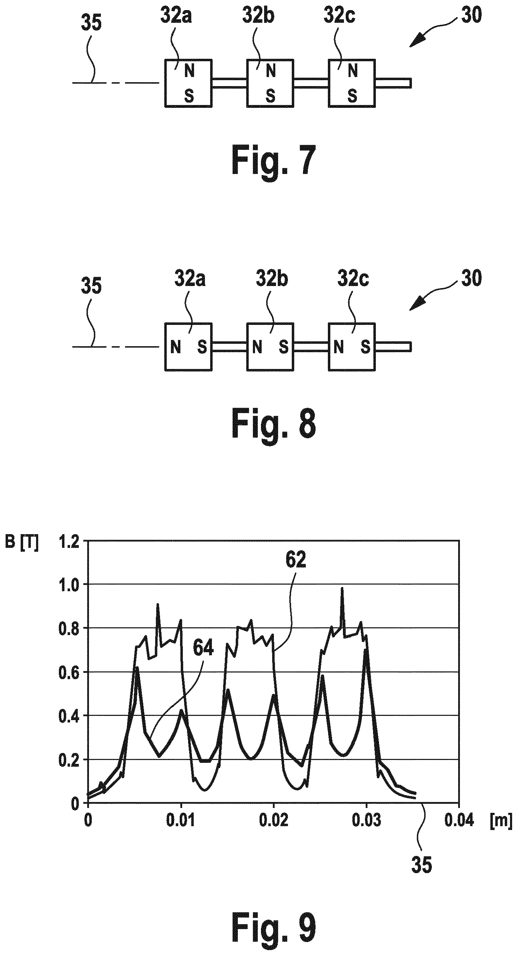

Figs. 7 and 8 show further aspects of the working element 30 which are also applicable to the working elements 50 and 60.

Fig. 7 shows that the magnetically acting elements 32a, 32b, 32c are magnetized in a direction perpendicular to the working element axis 35. For each magnetically acting element 32a, 32b, 32c, the magnetic poles N and S are illustrated in Fig. 7. "Magnetized in direction perpendicular to the working element axis 35" means that the pole axes from the S to the N pole are oriented perpendicular to the working element axis 35.

Fig. 8 shows an alternative to Fig. 7, where the magnetically acting elements 32a, 32b, 32c are magnetized in a direction parallel to the working element axis 35.

Fig. 9 shows a diagram illustrating, as an example, the magnetic flux density B (in Tesla) along the working element axis 35 (in meters) based on a FEM simulation for three magnetically acting elements made of neodymium iron boron (NdFeB). In this example, each magnetically acting element has an outer diameter of 5 mm and an inner diameter of 1.1 mm and a length of 5 mm. The grade of each magnet is N52. A first curve 62 shows the magnetic flux density B along the working element axis 35 for a magnetization perpendicular to the working element axis 35, and a curve 64 shows the magnetic flux density B for a magnetization parallel to the working element axis 35. Fig. 9 demonstrates that perpendicularly magnetized magnetically acting elements 32a, 32b, 32c (Fig. 7) have a higher magnetic flux density than parallely magnetized magnetically acting elements 32a, 32b, 32c (Fig. 8).

It can also be taken from Fig. 9 that the magnetic flux density B in the gaps (gaps 48a, 48b in Fig. 2) is much lower than around the magnetically acting elements 32a, 32b, 32c themselves. This provides much higher magnetic gradients at the edges of the magnetically acting elements 32a, 32b, 32c, thus generates larger magnetic forces due to higher magnetic gradients. Also, by reducing the gap width between single magnetically acting elements, the magnetic flux density can be made more homogeneous and concentrated over a smaller distance along the working element axis 35, while increasing the gap width will distribute the magnetic flux density over a larger distance along the working element axis 35.

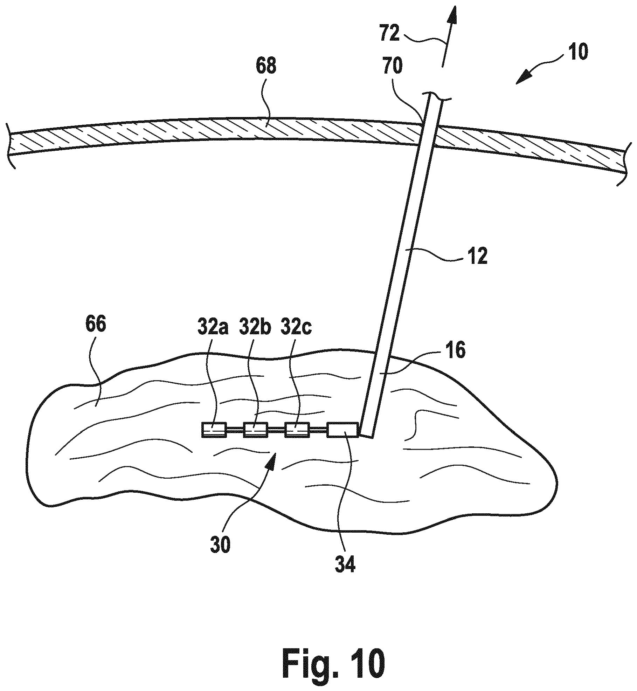

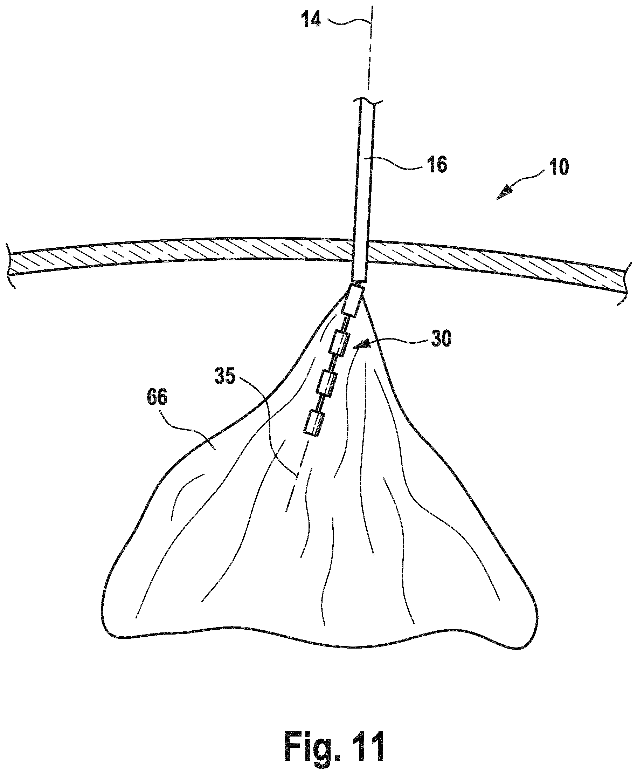

Figs. 10 and 11 show the medical instrument 10 (partially shown in Figs. 10 and 11) in a method for retracting tissue 66 by magnetic forces. The tissue 66 previously has been injected with a magnetic medium, for example stainless steel micro-particles suspension with glycerol/PBS fluid, iron-oxide nanoparticle suspensions, or a magnetic glue, e.g. cyanoacrylate-based magnetic glue (fluid) has been applied to the surface of the tissue 66. The shaft 12 of the instrument 10 is introduced through the body surface 68 through a small incision or access port 70 into which a trocar sleeve has been inserted (not shown) as usual in minimally invasive surgery. The trocar sleeve then serves as the access port for the medical instrument 10. The injection of the magnetic medium into the tissue 66 may have been performed through the same access port 70.

The working element 30 is fed close to or brought into contact with the surface of the tissue 66. To this end, the working element 30 is moved into an approximately 90° pivoting position with respect to the shaft 12, for example by moving the movable grip part 24, accordingly. Due to the magnetic attraction between the magnetic medium injected into the tissue 66 and the magnetically acting elements 32a, 32b, 32c, the surface of the tissue 66 adheres to the working element 30. In this way, each of the magnetically acting elements 32a, 32b, 32c is engaged with the magnetized tissue 66.

Upon withdrawing the medical instruments 10 in direction of an arrow 72 in Fig. 10, the tissue 66 is retracted as shown in Fig. 11. During withdrawal of the instrument 10, the working element 30 can freely pivot with respect to the shaft axis 14. Upon progressing withdrawal of the instrument 10, the pivoting angle of the working element 30 with respect to the shaft will progressively decrease to a small pivoting angle as shown in Fig. 11. It is to be understood that the angle of the working element 30 with respect to the shaft 12 could be as small as 0°. In fact, the maximal tissue retraction force, before the tissue 66 detaches from the working element 30, is significantly higher if the working element 30 is retracted parallel to the working element axis 35 than in case the working element 30 is retracted perpendicular to the working element axis 35. Thus, if the instrument 10 is withdrawn with the working element 30 remaining in its angular position as shown in Fig. 10, the tissue 66 would detach from the working element 30 earlier than in case the working element 30 has an angular orientation as shown in Fig. 11, i.e. is withdrawn approximately parallel to its axis 35.

In particular, when the tissue 66 is a soft tissue, some tissue is trapped within the gaps (gaps 48a, 48b in Fig. 2) between the magnetically acting elements 32a, 32b, 32c. In this case, when the working element 30 is retracted approximately parallel to its axis 35, a high tissue friction force is obtained in addition to the total magnetic attraction force. In contrast, when tissue is retracted with the working element 30 perpendicular to the axis 35, there is very little tissue friction force. This means, a larger maximal retraction force in a mode where the working element 30 is withdrawn approximately parallel to its axis 35 as shown in Fig. 11 can be used for reliable tissue manipulation, while the smaller maximal retraction force in the mode where the working element 30 is withdrawn approximately perpendicular to its axis 35 as shown in Fig. 10 (with the perpendicular orientation of the working element 30 with respect to the shaft being locked) can be used to facilitate the detachment of the tissue 66 from the working element 30 after completion of the surgical procedure. A variable retraction force may be obtained by changing and locking the working element in different angles between 0° to 90°.

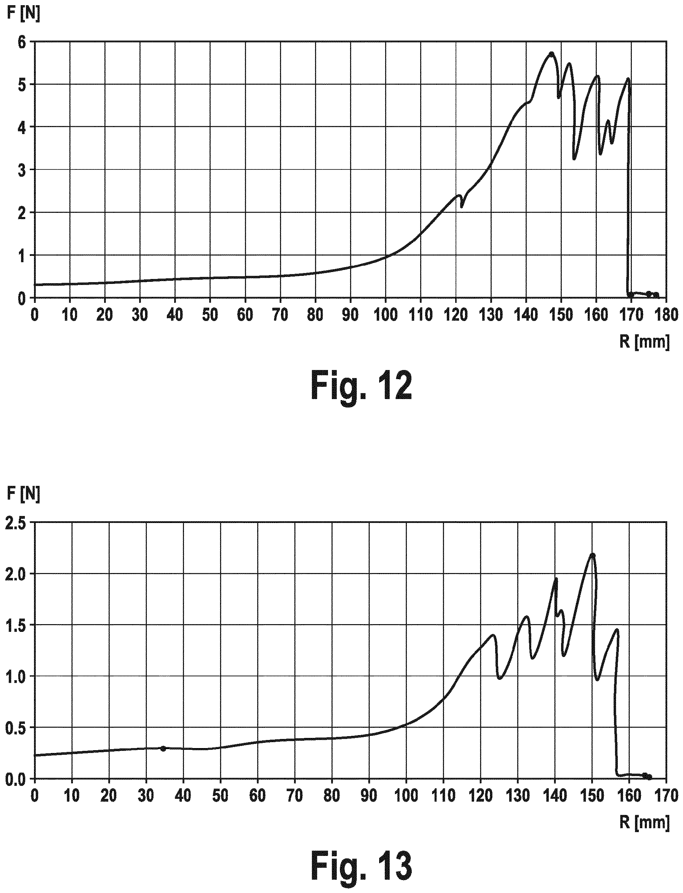

Figs. 12 through 14 show diagrams obtained in experiments which demonstrate effects described above.

Fig. 12 shows a diagram illustrating the retraction force F (in Newton) versus the retraction distance R (in millimeters) when the working element is withdrawn approximately parallel ("0°"-mode) to its longitudinal working element axis, and Fig. 13 shows the corresponding curve in case the working element is withdrawn approximately perpendicular ("90°"-mode, locked) to its axis.

The curves in Figs. 12 and 13 have been obtained by an experiment test in which an ex-vivo porcine bowel was magnetized by intraluminal injection of 2 ml magnetic fluid (0.5 g/ml stainless steel 410 micro-particles suspended into glycerol-PBS fluid). A working element with four magnetically acting elements of 10 mm outer diameter was used to retract the magnetized bowel in a mode where the working element is withdrawn parallel to its axis (Fig. 12), and in the mode where the working element was withdrawn perpendicular to its axis with the perpendicular orientation of the working element 30 with respect to the instrument shaft locked (Fig. 13).

A comparison between Figs. 12 and 13 demonstrates that retraction forces up to almost 6 Newton have been measured in the 0° mode, while the maximum retraction force in the 90° mode is less than 2,5 Newton.

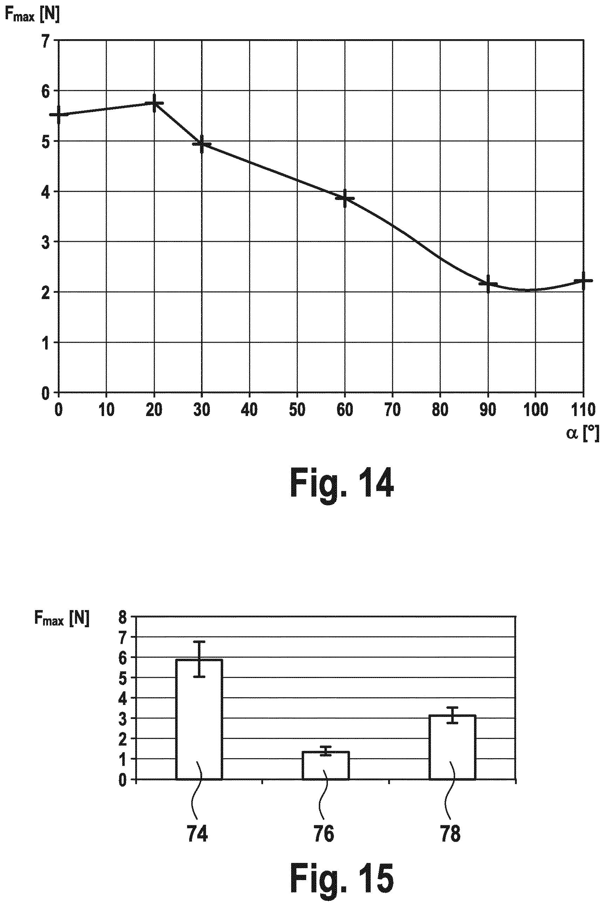

Fig. 14 shows a diagram illustrating the maximal retraction force Fₘₐₓ before detaching of the tissue in dependence on the angle α of the direction of the withdrawal of the working element with respect to the longitudinal working element axis, which corresponds to the angle of the longitudinal working element axis with respect to the instrument shaft axis. The resulting curve in Fig. 14 is based on the same experiment test as the curves in Figs. 12 and 13 with the working element fixed in different angular orientations with respect to the instrument shaft. As can be seen in Fig. 14, the maximal detachment force Fₘₐₓ at which the tissue detaches from the working element is highest for angles α in the range of 0° to 20°, and decreases with increasing angle α, and is lowest for angles in the range from 80° to 110°.

It has also been demonstrated by experiments that there is no significant difference between the "0°"-retraction mode with the working element 30 locked in the 0° position and a retraction mode where the working element is freely pivotable with respect to the shaft.

Fig. 15 shows results of a further experiment conducted with a magnetic phantom made of a surgical glove finger filled with 10 g stainless steel 410 micro-particles mixed with 2 ml glycerol/PBS fluid as a simulator for tissue or an organ. A working element similar to the working element 30 had six 2.5 mm diameter magnetically acting magnets made of NdFeB perpendicularly magnetized with respect to the longitudinal working element axis, each magnetically acting element had a length of 2.5 mm and an inner diameter of 1 mm through which a 1 mm rod or pin 38 passes, with a 2.5 mm gap width between the single magnetically acting elements. The grade of the magnets is N50. Fig. 15 shows the maximal retraction force Fₘₐₓ (in Newton) before detaching of the magnetic phantom from the working element for three different situations. Column 74 shows the maximal retraction force Fₘₐₓ obtained in a retraction mode where the working element is freely pivotable with respect to the instrument shaft, and column 76 shows the maximal retraction force Fₘₐₓ obtained with the working element angled by 90° and locked with respect to the shaft. Column 78 shows the maximal retraction force Fₘₐₓ obtained with a single 6 mm diameter magnetically acting element (also N50 grade) parallely magnetized. Fig. 15 demonstrates that a working element with a plurality of 2.5 mm magnetically acting elements like the magnetically acting elements 32a, 32b, 32c, 32d, 32e can generate much higher magnetic retraction forces than a working element having one 6 mm diameter magnetically acting element.

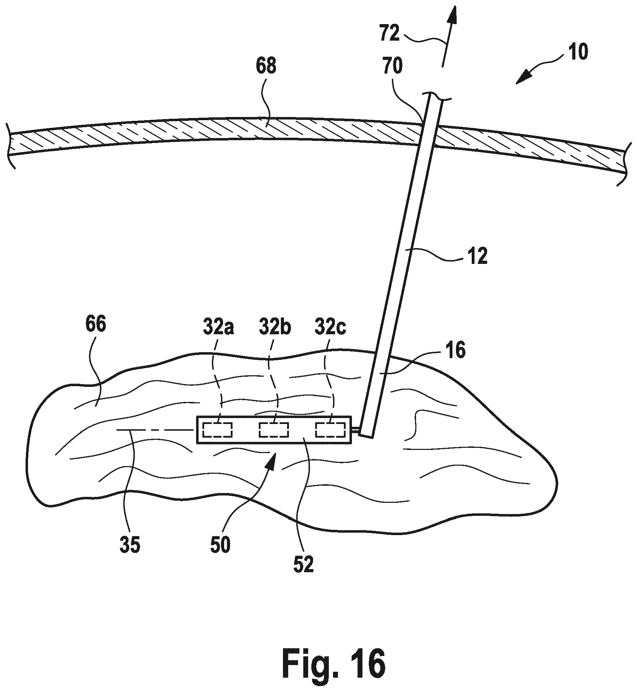

With reference to Figs. 16 through 20 and again reference to Fig. 5, another aspect will be described which deals with facilitating the detachment of tissue from the working element. Even in the "90°"-retraction mode by means of a working element like working element 30 according to Figs. 2 to 4, the detachment force can be too high for easily detaching or releasing the tissue from the working element. In case where more actively controlled or smooth release of the tissue is needed, the working element 50 in Fig. 5 should be used in the medical instrument 10 in Fig. 1.

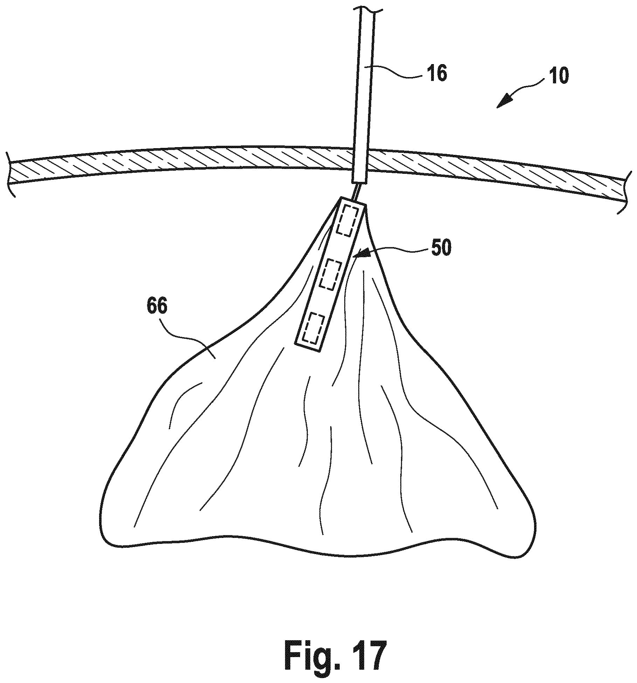

Similar to Fig. 10, Fig. 16 shows the medical instrument 10 (partially shown), now with the working element 50 arranged at the distal end portion 16 of the shaft 12 and introduced through the access port 70 in the body surface 68 and brought into contact with the magnetized tissue 66. The working element 50 is oriented with respect to the tissue 66 such that the exposed area of the magnetically acting elements 32a, 32b, 32c or, in other words, the opening 54 of the housing 52 faces the surfaces of the tissue 66. In this rotational orientation of the working element 50 with respect to its working element axis 35 the magnetic attraction force between the tissue 66 and the working element 50 is maximum. Here, the magnetically acting elements 32a, 32b, 32c are indicated by phantom lines because they are covered by the backside of the housing 52 in this rotational orientation with respect to the working element axis 35. Upon withdrawing the medical instrument 10 according to arrow 72, the tissue 66 is retracted as shown in Fig. 17, very similar as described with reference to Fig. 11.

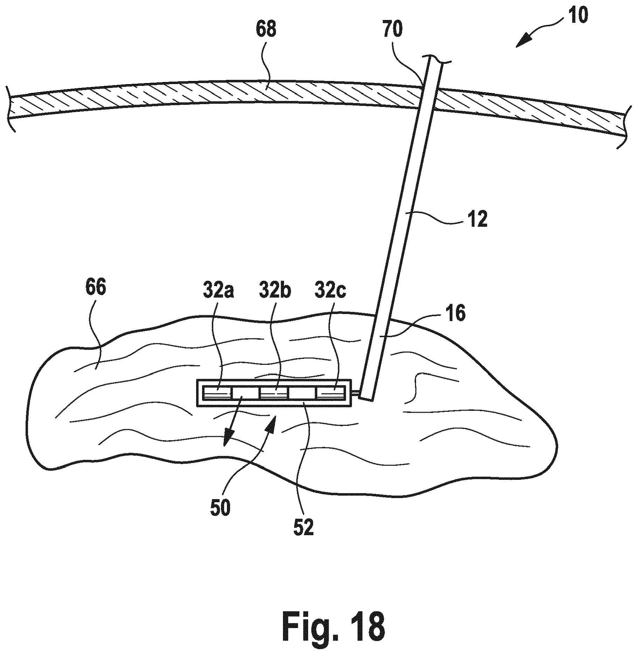

When the surgical procedure is finished, the working element 50 has to be detached from the tissue 66. Controlled or smooth detachment of the working element 50 is easily accomplished by rotating the working element 50 about the working element axis 35, until the housing 52, in more detail the backside of the housing 52, faces the surface of the tissue 66 as shown in Fig. 18. Since the housing 52 is magnetically inactive or at least magnetically weaker active than the magnetically acting elements 32a, 32b, 32c, the magnetic attraction force between the tissue 66 and the working element 30 is reduced to such an extent that the working element 50 can be smoothly detached from the tissue 66.

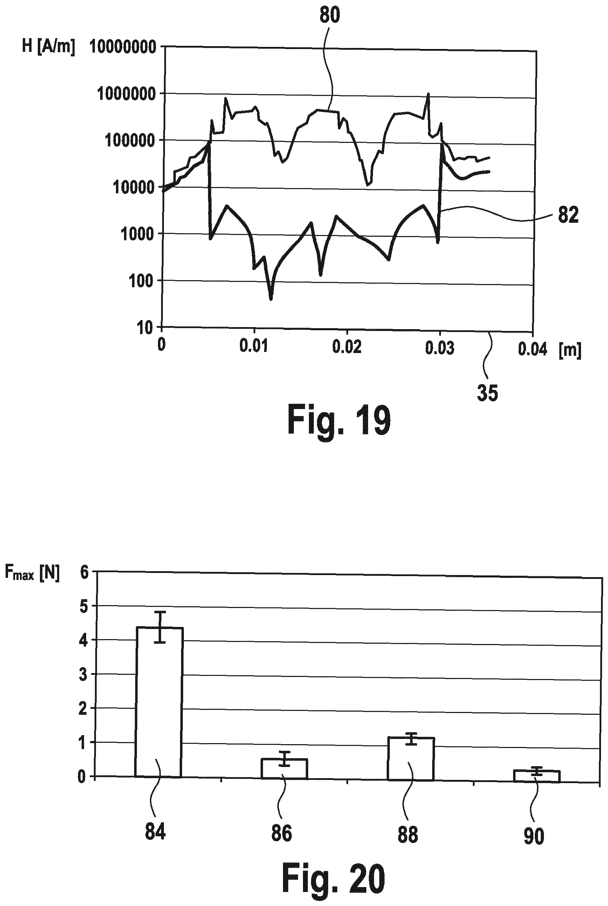

Fig. 19 shows the magnetic field H (in Ampere/meter) along the working element axis 35, wherein a curve 80 shows the magnetic field H on the exposed side of the magnetically acting elements 32a, 32b, 32c, while a curve 82 shows the magnetic field H on the backside of the housing 52 along the working element axis 35. As can be taken from Fig. 19, the magnetic field is much weaker, by orders of magnitude, on the backside of the housing 52 than on the exposed side of the magnetically acting elements 32a, 32b, 32c.

In experiments, a working element like the working element 50 in Fig. 5 has been used for retracting a magnetized bowel segment as the tissue 66. The working element had three magnetically acting elements with 4 mm diameter and 4 mm length which were parallely magnetized. The grade of the magnetically acting elements was N45. The housing was made of stainless steel.

Measurements of the maximal retraction force Fₘₐₓ before detachment of the tissue from the working element were conducted for several retraction modes. In a first retraction mode, the working element was freely pivotable with respect to the instrument shaft axis with the exposed side of the magnetically acting elements 32a, 32b, 32c facing the tissue 66. The second retraction mode was like the first mode, but now with the closed backside of the housing facing the tissue 66. The third mode differed from the first mode in that the working element was oriented at an angle of 90° with respect to the instrument shaft axis and locked in that position. The fourth retraction mode was like the second retraction mode, but now with the working element being in a 90° orientation with respect to the instrument shaft axis.

Fig. 20 shows the experimental results of the maximal retraction force Fₘₐₓ for the first retraction mode (column 84), the second retraction mode (column 86), the third retraction mode (column 88), and the fourth retraction mode (column 90). Fig. 20 demonstrates that high and sufficient retraction force can be obtained with the working element 50 being freely pivotable with the exposed area of the magnetically acting elements 32a, 32b, 32c facing the tissue 66 (column 84), namely a maximal retraction force of more than 4 Newton, while the detachment force for detaching the working element 50 from the tissue 66 is very low if the working element 50 is rotated such that the backside of the housing 52 faces the tissue (columns 86 and 90).

Thus, the working element 50 does not only ensure a high retraction force for retracting tissue or an organ, but also facilitates the detachment of the working element 50 from the tissue 66 after finish of the surgical procedure.

## Claims

1. A medical instrument for manipulating, in particular retracting tissue or an organ in the human or animal body, comprising an elongated shaft (12) having a distal end portion (16), and a working element (30; 50; 60) arranged at the distal end portion (16) of the shaft (12), wherein the working element (30; 50; 60) has at least one magnetically acting element (32a) generating a magnetic field for manipulating the tissue or organ, wherein the working element (30; 50; 60) has a plurality of magnetically acting elements (32a, 32b, 32c; 32d, 32e) each generating a magnetic field, wherein the magnetically acting elements (32a, 32b, 32c; 32d, 32e) are arranged in-line along a longitudinal working element axis (35) of the working element (30; 50; 60), **characterized in that** the magnetically acting elements (32a, 32b, 32c; 32d, 32e) are adjustable in position along the longitudinal working element axis (35).

2. The instrument of claim 1, **characterized in that** the magnetically acting elements (32a, 32b, 32c) are spaced apart from one another, with a gap (48a, 48b) between adjacent magnetically acting elements (32a, 32b, 32c).

3. The instrument of claim 1, **characterized in that** the magnetically acting elements (32a, 32b, 32c 32d, 32e)are arranged without a gap between the magnetically acting elements.

4. The instrument of any one of claims 1 through 3, **characterized in that** the magnetically acting elements (32a, 32b, 32c; 32d, 32e) are magnetized in a direction perpendicular to the longitudinal working element axis (35).

5. The instrument of any one of claims 1 through 3, **characterized in that** the magnetically acting elements (32a, 32b, 32c; 32d, 32e) are magnetized in a direction parallel to the longitudinal working element axis (35).

6. The instrument of any one of claims 1 through 5, **characterized in that** the working element (30; 60) has a thin rod or pin (38) and the magnetically acting elements (32a, 32b, 32c; 32d, 32e) each have a bore through which the rod or pin (38) passes.

7. The instrument of any one of claims 1 through 6, **characterized in that** the working element (50) has a housing (52) magnetically inactive or magnetically weaker active than the magnetically acting elements (32a, 32b, 32c), in which the magnetically acting elements (32a, 32b, 32c) are accommodated, wherein the housing (52) has a lateral opening (54) which is limited in circumferential direction around the longitudinal working element axis (35) so that the magnetically acting elements (32a, 32b, 32c) are only partially exposed.

8. The instrument of any one of claims 1 through 7, **characterized in that** the working element (30; 50; 60) is pivotably connected to the distal end portion (16) of the shaft (12) so that an angle of the longitudinal working element axis (35) with respect to a longitudinal shaft axis (14) of the shaft (12) can be changed.

9. The instrument of claim 8, **characterized in that** the angle of the longitudinal working element axis (35) with respect to the shaft axis (14) is changeable from about 0° to at least 90°.

10. The instrument of claim 8 or 9, **characterized in that** the working element (30; 50; 60) can be fixed in a set angular pivoting position, and/or the working element (30; 50; 60) can be released for free pivoting movement.

11. The instrument of any one of claims 8 through 10, **characterized in that** the working element (30; 50; 60) is connected to a movable grip part (24) of a handle (20) arranged at a proximal end portion (18) of the shaft (12) for pivoting the working element (30; 50; 60).

12. The instrument of any one of claims 1 through 11, **characterized in that** the working element (50) is rotatable about the longitudinal working element axis (35).

13. The instrument of any one of claims 1 through 12, **characterized in that** the plurality of magnetically acting elements (32a, 32b, 32c; 32d, 32e) has at least three magnetically acting elements (32a, 32b, 32c; 32d, 32e).

14. The instrument of any of claims 1 through 13, **characterized in that** the magnetically acting elements (32a, 32b, 32c; 32d, 32e) each have a maximal size perpendicular to the longitudinal working element axis (14) which is equal to or smaller than a diameter (Dₛ) of the shaft.

## Patentansprüche

1. Medizinisches Instrument zum Manipulieren, insbesondere Zurückziehen von Gewebe oder eines Organs im menschlichen oder tierischen Körper, mit einem langerstreckten Schaft (12), der einen distalen Endabschnitt (16) aufweist, und einem Arbeitselement (30¸50; 60), das an dem distalen Endabschnitt (16) des Schaftes (12) angeordnet ist, wobei das Arbeitselement (30; 50; 60) zumindest ein magnetisch wirkendes Element (32a) aufweist, das ein Magnetfeld zum Manipulieren des Gewebes oder Organs erzeugt, wobei das Arbeitselement (30; 50; 60) eine Mehrzahl an magnetisch wirkenden Elementen (32a, 32b, 32c; 32d, 32e) aufweist, die jeweils ein Magnetfeld erzeugen, wobei die magnetisch wirkenden Elemente (32a, 32b, 32c; 32d, 32e) in Reihe entlang einer längsverlaufenden Arbeitselementachse (35) des Arbeitselements (30; 50; 60) angeordnet sind, **dadurch gekennzeichnet, dass** die magnetisch wirkenden Elemente (32a 32b, 32c; 32d, 32e) in ihrer Position entlang der längsverlaufenden Arbeitselementachse (35) einstellbar sind.

2. Instrument nach Anspruch 1, **dadurch gekennzeichnet, dass** die magnetisch wirkenden Elemente (32a, 32b, 32c) durch eine Lücke (48a, 48b) zwischen benachbarten magnetisch wirkenden Elementen (32a, 32b, 32c) voneinander beabstandet sind.

3. Instrument nach Anspruch 1, **dadurch gekennzeichnet, dass** die magnetisch wirkenden Elemente (32a, 32b, 32c; 32d, 32e) ohne eine Lücke zwischen den magnetisch wirkenden Elementen angeordnet sind.

4. Instrument nach einem der Ansprüche 1 bis 3, **dadurch gekennzeichnet, dass** die magnetisch wirkenden Elemente (32a, 32b, 32c; 32d, 32e) in einer Richtung senkrecht zur längsverlaufenden Arbeitselementachse (35) magnetisiert sind.

5. Instrument nach einem der Ansprüche 1 bis 3, **dadurch gekennzeichnet, dass** die magnetisch wirkenden Elemente (32a, 32b, 32c; 32d, 32e) in einer Richtung parallel zur längsverlaufenden Arbeitselementachse (35) magnetisiert sind.

6. Instrument nach einem der Ansprüche 1 bis 5, **dadurch gekennzeichnet, dass** das Arbeitselement (30; 50; 60) einen dünnen Stab oder Stift (38) aufweist und die magnetisch wirkenden Elemente (32a, 32b, 32c; 32d, 32e) jeweils eine Bohrung aufweisen, durch die der Stab oder Stift (38) hindurchgeht.

7. Instrument nach einem der Ansprüche 1 bis 6, **dadurch gekennzeichnet, dass** das Arbeitselement (50) ein Gehäuse (52) aufweist, das magnetisch unwirksam oder magnetisch schwächer wirksam ist als die magnetisch wirkenden Elemente (32a, 32b, 32c), in dem die magnetisch wirkenden Elemente (32a, 32b, 32c) aufgenommen sind, wobei das Gehäuse (52) eine seitliche Öffnung (54) aufweist, die in Umfangsrichtung um die längsverlaufende Arbeitselementachse (35) begrenzt ist, sodass die magnetisch wirkenden Elemente (32a, 32b, 32c) nur teilweise frei liegen.

8. Instrument nach einem der Ansprüche 1 bis 7, **dadurch gekennzeichnet, dass** das Arbeitselement (30; 50; 60) verschwenkbar mit dem distalen Endabschnitt (16) des Schaftes (12) verbunden ist, so dass ein Winkel der längsverlaufenden Arbeitselementachse (35) bezüglich einer längsverlaufenden Schaftachse (14) des Schaftes (12) verändert werden kann.

9. Instrument nach Anspruch 8, **dadurch gekennzeichnet, dass** der Winkel der längsverlaufenden Arbeitselementachse (35) bezüglich der Schaftachse (14) von etwa 0° bis zumindest 90° veränderbar ist.

10. Instrument nach Anspruch 8 oder 9, **dadurch gekennzeichnet, dass** das Arbeitselement (30; 50; 60) in einer eingestellten Winkel- Schwenkposition fixiert werden kann, und/oder das Arbeitselement (30; 50; 60) zur freien Schwenkbewegung freigegeben werden kann.

11. Instrument nach einem der Ansprüche 8 bis 10, **dadurch gekennzeichnet, dass** das Arbeitselement (30: 50; 60) mit einem beweglichen Griffteil (24) eines Handgriffs (20), der an einem proximalen Endabschnitt (18) des Schaftes (12) angeordnet ist, zum Verschwenken des Arbeitselementes (30; 50; 60) verbunden ist.

12. Instrument nach einem der Ansprüche 1 bis 11, **dadurch gekennzeichnet, dass** das Arbeitselement (50) um die längsverlaufende Arbeitselementachse (35) drehbar ist.

13. Instrument nach einem der Ansprüche 1 bis 12, **dadurch gekennzeichnet, dass** die Mehrzahl an magnetisch wirkenden Elementen (32a, 32b, 32c; 32d, 32e) zumindest drei magnetisch wirkende Elemente (32a, 32b, 32c; 32d, 32e) aufweist.

14. Instrument nach einem der Ansprüche 1 bis 13, **dadurch gekennzeichnet, dass** die magnetisch wirkenden Elemente (32a, 32b, 32c; 32d, 32e) jeweils eine maximale Größe senkrecht zu der längsverlaufenden Arbeitselementachse (14) aufweisen, die gleich einem oder kleiner als ein Durchmesser (Dₛ) des Schaftes ist.

## Revendications

1. Instrument médical pour manipuler, en particulier pour rétracter un tissu ou un organe dans le corps humain ou animal, comprenant un arbre allongé (12) ayant une partie d'extrémité distale (16), et un élément de travail (30 ; 50 ; 60) agencé au niveau de la partie d'extrémité distale (16) de l'arbre (12), où l'élément de travail (30; 50; 60) a au moins un élément à action magnétique (32a) générant un champ magnétique pour manipuler le tissu ou l'organe, où l'élément de travail (30 ; 50 ; 60) a une pluralité d'éléments à action magnétique (32a, 32b, 32c ; 32d, 32e) générant chacun un champ magnétique, où les éléments à action magnétique (32a, 32b, 32c ; 32d, 32e) sont agencés en série le long d'un axe d'élément de travail longitudinal (35) de l'élément de travail (30 ; 50 ; 60), **caractérisé en ce que** les éléments à action magnétique (32a, 32b, 32c ; 32d, 32e) sont réglables en position le long de l'axe d'élément de travail longitudinal (35).

2. Instrument de la revendication 1, **caractérisé en ce que** les éléments à action magnétique (32a, 32b, 32c) sont espacés les uns des autres, avec un espace (48a, 48b) entre des éléments à action magnétique adjacents (32a, 32b, 32c).

3. Instrument de la revendication 1, **caractérisé en ce que** les éléments à action magnétique (32a, 32b, 32c ; 32d, 32e) sont agencés sans espace entre les éléments à action magnétique.

4. Instrument de l'une quelconque des revendications 1 à 3, **caractérisé en ce que** les éléments à action magnétique (32a, 32b, 32c ; 32d, 32e) sont magnétisés dans une direction perpendiculaire à l'axe d'élément de travail longitudinal (35).

5. Instrument de l'une quelconque des revendications 1 à 3, **caractérisé en ce que** les éléments à action magnétique (32a, 32b, 32c ; 32d, 32e) sont magnétisés dans une direction parallèle à l'axe d'élément de travail longitudinal (35).

6. Instrument de l'une quelconque des revendications 1 à 5, **caractérisé en ce que** l'élément de travail (30 ; 60) a une tige ou une broche mince (38) et les éléments à action magnétique (32a, 32b, 32c ; 32d, 32e) ont chacun un alésage à travers lequel la tige ou la broche (38) passe.

7. Instrument de l'une quelconque des revendications 1 à 6, **caractérisé en ce que** l'élément de travail (50) a un boîtier (52) magnétiquement inactif ou magnétiquement moins actif que les éléments à action magnétique (32a, 32b, 32c), dans lequel les éléments à action magnétique (32a, 32b, 32c) sont logés, où le boîtier (52) a une ouverture latérale (54) qui est limitée dans la direction circonférentielle autour de l'axe d'élément de travail longitudinal (35) de sorte que les éléments à action magnétique (32a, 32b, 32c) ne sont que partiellement exposés.

8. Instrument de l'une quelconque des revendications 1 à 7, **caractérisé en ce que** l'élément de travail (30 ; 50 ; 60) est relié en pivotement à la partie d'extrémité distale (16) de l'arbre (12) de sorte qu'un angle de l'axe d'élément de travail longitudinal (35) par rapport à un axe d'arbre longitudinal (14) de l'arbre (12) peut être changé.

9. Instrument de la revendication 8, **caractérisé en ce que** l'angle de l'axe d'élément de travail longitudinal (35) par rapport à l'axe d'arbre (14) peut être changé d'environ 0° à au moins 90°.

10. Instrument de la revendication 8 ou 9, **caractérisé en ce que** l'élément de travail (30 ; 50 ; 60) peut être fixé dans une position de pivotement angulaire définie, et/ou l'élément de travail (30 ; 50 ; 60) peut être libéré pour effectuer un mouvement de pivotement libre.

11. Instrument de l'une quelconque des revendications 8 à 10, **caractérisé en ce que** l'élément de travail (30 ; 50 ; 60) est relié à une partie de préhension mobile (24) d'une poignée (20) agencée au niveau d'une partie d'extrémité proximale (18) de l'arbre (12) pour faire pivoter l'élément de travail (30 ; 50 ; 60).

12. Instrument de l'une quelconque des revendications 1 à 11, **caractérisé en ce que** l'élément de travail (50) peut tourner autour de l'axe d'élément de travail longitudinal (35).

13. Instrument de l'une quelconque des revendications 1 à 12, **caractérisé en ce que** la pluralité d'éléments à action magnétique (32a, 32b, 32c ; 32d, 32e) a au moins trois éléments à action magnétique (32a, 32b, 32c ; 32d, 32e).

14. Instrument de l'une des revendications 1 à 13, **caractérisé en ce que** les éléments à action magnétique (32a, 32b, 32c ; 32d, 32e) ont chacun une taille maximale perpendiculaire à l'axe d'élément de travail longitudinal (14) qui est inférieure ou égale à un diamètre (Dₛ) de l'arbre.
